Europäisches Patentamt

European Patent Office

Office européen des brevets

(19)

(11) Publication number: **0 083 497**
A2

(12) # EUROPEAN PATENT APPLICATION

(21) Application number: **82306883.8**

(22) Date of filing: **23.12.82**

(51) Int. Cl.³: **A 61 K 39/35**, A 61 K 39/36

(30) Priority: **06.01.82 GB 8200329**

(43) Date of publication of application: **13.07.83**
**Bulletin 83/28**

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Moran, David Martin, 1 Durnsford Way, Cranleigh Surrey (GB)**

(74) Representative: **Russell, Brian John et al, European Patent Attorney Beecham Pharmaceuticals Great Burgh Yew Tree Bottom Road, Epsom Surrey, KT18 5XQ (GB)**

(54) Process for preparing modified allergens, their use in treating allergic conditions.

(57) A process for preparing a modified allergen comprises protecting a substantial proportion of the allergenic sites, reacting the allergen so protected with a cross-linking agent to give a linked product of substantially enhanced molecular weight distribution, and then deprotecting the linked product. The modified allergen produced by this process is useful in the treatment of allergic condition.

EP 0 083 497 A2

## Process for preparing Modified Allergens, and their use in treating allergic conditions

The present invention relates to a process for preparing modified allergens, and to the use of the modified allergens in the treatment of allergic conditions.

It is well known that some individuals are allergic or hypersensitive to certain allergenic materials such as pollens, house dust, cat fur, cereals and a host of other common substances. Such individuals can suffer acute discomfort as a result of their allergic conditions which may manifest themselves in such diseases as asthma, hay fever, eczema, dermatitis and migraine.

It is thus desirable to immunise or desensitise such individuals against the given allergen by using its natural antigenicity to build up protective antibodies in the patient, whilst at the same time minimising the concomitant allergenicity.

It is known to modify allergenic material to reduce its allergenicity relative to its desensitising and/or immunogenic properties by intermolecular linkage with inter alia a polyaldehyde, a polyketone, a carbodiimide, an epihalohydrin or an inorganic cyanate.

However, it is also believed that the immunogenicity of such an immunogenic species is a function of the number of its antigenic sites. These in turn are known to be sensitive to changes in the chemical nature of the moieties contributing to each antigenic structure. Thus, although the above linkage represents an advance in the art, a disadvantage is that some of the antigenic sites are modified in intermolecular linkage, whilst others are lost, or modified, in inevitable concomitant intramolecular linkage.

A method of overcoming, or lessening, this disadvantage, has now been found.

Accordingly, this invention provides a process for the preparation of a modified allergen which process comprises protecting a substantial proportion of the allergenic sites, reacting the allergen so protected with a cross linking agent to give a linked product of substantially enhanced molecular weight distribution, and then deprotecting the linked product.

The term allergenic sites, as used herein, means those structural elements which combine with human anti-native allergen IgE antibody and/or anti-native allergen antibodies of other isotypes.

The allergenic starting material which is modified according to the process of this invention may be obtained from an allergen-containing substance such as pollen by extracting the allergen-containing substance with a suitable solvent, usually aqueous, in a known manner. The allergenic extract obtained in this way consists principally of protein or glycoprotein, usually contaminated with free carbohydrate. The allergenic extract is then usually purified by removing some of the contaminants, e.g. by dialysis, precipitation or gel filtration, and the resulting

- 3 -

0083497

purified allergenic material may then be treated
according to the process of invention. A full
description of some of the techniques available can be
found in an article by J.N. Newell in the Journal of
Allergy, Vol. 13, 1942, pages 177 to 203, particularly
page 187. In another useful extraction procedure, the
allergen-containing material or an aqueous extract
thereof is treated with aqueous phenol and the
allergenic extract is recovered from the phenol phase.

It will be appreciated that it is of course
necessary to leave unprotected sufficient chemical
functional groups on the allergen in the process of the
invention to allow the required linking reaction.
While no precise values are generally applicable, by
way of a guide it is believed that at least 10%, more
suitably 10% to 80%, and most suitably 50 to 80% of the
chemical functional groups should be left unprotected.
Thus it follows that 90%, more suitably 20% to 90%, and
most suitably 20 to 50% of the chemical functional
groupswill be protected.

Suitable protecting groups for the practice of the
present invention are those which are readily
reversible under mild reaction conditions, provided of
course that the deprotection results in the substantial
recovery of allergenic activity. The choice of
protecting groups will be apparent to those skilled in
the art, but the following reagents may be mentioned as
providing suitable protecting groups: unsaturated
dicarboxylic acid anhydrides, trifluoroacetic
anhydride, ethyl thio trifluoroacetate, ethoxyformic
anhydride, diketene, $\alpha$-hydroxy aldehydes or ketones.

Preferred dicarboxylic acid anhydrides are maleic
anhydride, methyl maleic anhydride, dimethyl maleic
anhydride and exo cis 3,6-endoxo $\Delta^4$ tetra- or hexa-

hydrophthalic anhydride. Preferred $\alpha$-hydroxy aldehydes or ketones are glycolaldehyde or acetol.

In a preferred embodiment of the process of the invention, the protecting reaction is carried out by treating the extracted allergen material with an acid anhydride, in aqueous medium in the presence of a buffer, for example a borate buffer.

The reaction mixture is preferably maintained at a weakly alkaline pH at ambient temperature for up to five or six hours, and the acylated product recovered by dialysis and freeze drying.

The proportion of chemical functional groups on the allergen which are protected can be controlled by adjusting the concentrations of allergen and/or protecting agent in the reaction mixture and/or by controlling the reaction conditions. The choice of concentrations and reaction conditions is a matter of routine trial and error which should present little difficulty to those skilled in the art.

The protected allergen prepared as described above can then be reacted with the crosslinking agent in conventional manner, as for example described in U.K. Patent No. 1,282,163.

The cross linking agent may be any of those disclosed in U.K. Patent No. 1,282,163, but a polyaldehyde is preferred. The polyaldehydes for use include dialdehydes and other higher polyaldehydes. Generally, of the polyaldehydes which may be used we prefer to use dialdehydes, having from 2 to 24 carbon atoms in the molecule. The dialdehyde may be straight or branched aliphatic, cycloaliphatic, aromatic or heterocyclic. Glutaraldehyde (i.e. pentanedial), butanedial, and dialdehydes, preferably having from 4 to 24 carbon atoms, are amongst suitable dialdehydes. A particularly preferred dialdehyde is glutaraldehyde.

When dialdehydes are employed it is preferred to operate at a temperature below 37°C, more preferably at ambient temperature. When employing dialdehydes, extreme reaction conditions should be avoided in order to reduce undesirable by-products such as are produced by self condensation of the aldehydes.

In the case of polyaldehydes, the pH at which the process should be carried out is not critical. A suitable pH range is from 4 to 8, with a pH of substantially 7 being preferred. Very low pH values should be avoided in order to reduce possible self condensation of the aldehydes.

After the cross linking reaction, the protecting groups on the allergen are removed in any convenient manner. This deprotection process may occur under alkaline or mildly acidic conditions at ambient or slightly above ambient temperature. The process may take up to several hours, but it is preferred to leave the reaction mixture for an overnight period to ensure full deprotection. The appropriate acidic conditions may be achieved by adjusting the pH of the buffered, protected allergen material to about 5 to 6 by addition of dilute mineral acid, such as HCl.

The resultant modified allergen is a polymerised allergen material having therapeutic activity. The degree of polymerisation and molecular weight of the material will vary according to the nature of the allergen. For example, a polymerised grass pollen extract could have a degree of polymerisation from about 5 up to about 200, and a molecular weight from about 200,000 to about 20,000,000.  The distribution of molecular weights in a given sample of polymerised material will vary according to the nature of the allergen.

The invention also provides a pharmaceutical composition comprising a polymerised allergen prepared by the process described above, and a pharmaceutically acceptable carrier.

Such compositions may be in the form of nasal and oral liquid preparations, powders, granules and reconstitutable powders; and injectable reconstitutable powders and fluid suspensions.  The compositions may also be in the form of adsorbates employing L-tyrosine or alum as the adsorbent phase, prepared according to the general method disclosed in U.K. Patent Specification No. 1,377,074.

A preferred composition of the invention is an injectable desensitisation vaccine.

Usually a patient receiving treatment with such vaccines is administered a number of injections, spread over a period of weeks or days, each at an increasing dosage leval of allergen until the patient is desensitised such that the allergic reaction to the allergen is reduced or eliminated.

It will be appreciated that the dose of active material contained in the compositions of the invention will depend in the usual way on factors such as the allergen used, the sensitivity of the allergic reaction of the patient, and the stage of therapy.  However, by way of illustration, suitably 1 to 10 000 PNU of allergen per dose will be used, doses towards the lower end of this scale for early in therapy, doses towards the higher end of this scale for later in therapy.

0083497

Such compositions are preferably in unit dosage forms, either liquid or in the form of reconstitutable powders for fluid forms.

For preparing injectables the modified allergen may suitably be sterilized by filtration. Suspension may be followed by filling into a vial or ampoule and sealing. Excipients such as a local anaesthetic, preservatives and buffering agents can also be dissolved in the vehicle. The particles of active agent should be small enough not to block the orifice of an injection needle.

Nasal and oral liquid preparations may be in the form of, for example, aqueous suspensions, or may be presented in a dry product for reconstitution with water or other suitable vehicle before use. Such liquid preparations may contain conventional additives such as suspending agents, emulsifying agents, non-aqueous vehicles, preservatives, and, if desired, conventional colouring agents.

Oral liquid preparations may also be in the form of oily suspensions or aqueous syrups or elixirs. Such liquid preparations may also include edible oils and conventional flavouring agents.

Dry solid compositions for nasal and oral administration may be in unit dose presentation form, and may contain conventional excipients such as fillers, disintegrants and acceptable wetting agents.

Nasal powders may be prepared by methods well known in normal pharmacy.

The following Examples illustrate the preparation of modified allergens in accordance with the present invention.

0083497.

Example 1

Protection and Deprotection of Timothy Pollen Extracts by Acetoacetylation

Timothy pollen extract was dissolved in borate buffer (0.05M, pH 8.6) at 5 mg/ml and treated with various amounts of diketene. The reaction was continued for 5 hours at ambient temperature with stirring and then dialysed exhaustively against ammonium bicarbonate (1 mM) at 4°C. The retentates were finally recovered by freeze drying. Characterisation of the acetoacetylated products obtained in terms of primary amino and skin test activity (as measured in grass pollen sensitive volunteers) is shown in Table 1.

Deprotection of the acylated functional group was achieved by treatment of the acetoacetylated product (2 mg/ml) in phosphate buffer (0.05M, pH 7) with hydroxylamine (4.8 mM) for an overnight period at ambient temperature. The products were dialysed extensively against ammonium bicarbonate (1 mM) at 4°C and the retentates were finally recovered by freeze drying. Characterisation of the products is shown in Table 1.

Table 1

Acetoacetylation and deacylation of Timothy grass
pollen extract

| Material* (code) | Diketene (μl) | Prior to treatment with $NH_2OH$ | | Post treatment with $NH_2OH$ | |
|---|---|---|---|---|---|
| | | $1°NH_2$ (meq/gm) | skin test+ activity (% control) | $1°NH_2$ (meq/gm) | skin test activity (% control) |
| Timothy extract | | | | | |
| (T1) | 0 | 0.53 | 95 | 0.73 | 97 |
| (T2) | 10 | 0.12 | 33 | - | - |
| (T3) | 20 | 0.09 | 20 | - | - |
| (T4) | 100 | 0.07 | 9 | 0.57 | 54 |
| (T5) | 200 | 0.08 | 6 | 0.45 | 50 |

\* 25 mg extract used for each reaction.

+ prick tests carried out in grass pollen sensitive
volunteers.

Example 2

Protection and Deprotection of Rye Grass Pollen
Extracts by Citraconylation

Rye grass pollen extract was dissolved in borate
buffer (0.1M, pH 8) at 20 mg/ml and stirred at room
temperature during the addition of citraconic anhydride
solution (2% v/v acetone); maintenance of the solution
at pH 8 was achieved by dropwise addition of sodium
hydroxide solution (1M). After 5 minutes the pH
remained constant and the solutions were left for a
further 30 minutes, then dialysed exhaustively against
$NH_4HCO_3$ (10mM) at 4°C and finally freeze dried.
Characterisation of the products obtained using varying
amounts of citraconic anhydride is shown in Table 2.

Deprotection of the acylated functional groups was
achieved by treatment of the citraconylated
preparations ($R_1$-$R_4$) in phosphate buffer (0.5M, pH 5.2;
adjustment of buffer from pH 7.0 was effected by
addition of dilute HCl) for an overnight period at
30°C. The products were dialysed extensively against
ammonium bicarbonate (10mM) at 4°C and the retentates
were recovered by freeze drying. Characterisation of
the products is shown in Table 2.

Table 2

Citraconylation and decitraconylation of rye grass pollen
extract

| Material* | Citraconic anhydride (μl) | Prior to treatment with acid | | Post deacylations | |
|-----------|---------------------------|------------------------------|-----------------|-------------------|-----------------|
|           |                           | $1°NH_2$ (meq/gm)            | Aller-genicity+ | $1°NH_2$ (meq/gm) | Aller-genicity+ |
| R1        | 0                         | 0.51                         | 0.6             | 0.58              | 0.6             |
| R2        | 60                        | 0.39                         | 0.3             | 0.54              | 0.8             |
| R3        | 160                       | 0.25                         | 3.0             | 0.55              | 0.5             |
| R4        | 250                       | 0.18                         | 28.0            | 0.53              | 4.0             |

\* 50 mg extract used for each reaction.
+ Concentration (μg/ml) required to achieve 50% inhibition
  of rye grass pollen extract specific IgE antibody
  (obtained from grass pollen sensitive individuals)
  binding to rye grass pollen extract covalently bonded
  to paper discs.

Example 3

Protection and Deprotection of House Dust Mite Extracts
(D. Pteronyssinus) by Citraconylation

    D. Pteronyssinus extract was dissolved in
phosphate buffer (0.1M, pH 8.0) at 2mg/ml and stirred
at ambient temperature during the addition of
citraconic anhydride solution (2.0% v/v in acetone or
aprotic solvents); maintenance of the solution at pH
8.0 was achieved by dropwise addition of sodium
hydroxide (1M). After 5 minutes, the pH remained
essentially constant and the solutions were left for a
further 30 minutes, then dialysed exhaustively against
phosphate buffer (0.01M, pH 8.0) or chromatographed
using a P2 Biogel column, prior equilibrated with the
dilute phosphate buffer; in the latter instance, the
excluded components were collected and pooled.

    Characterisation of products obtained (HD1 - HD6)
using various amounts of citraconic anhydride is shown
in Table 3.

    Deprotection of acylated functional group was
achieved by treatment of the citraconylated
preparations in phosphate buffer (0.01M) at various
mildly acidic pH values; adjustment of buffer from pH
8.0 was effected by dropwise addition of sodium
dihydrogen phosphate solution (1M). The extract
solutions were left for an overnight period at room
temperature and then basified with dilute sodium
hydroxide (1M) to pH 8.0.

    Characterisation data for products deprotected in
the above manner and at various pH and temperature
conditions are given in Table 3.

The data shows that allergenic activity of house
dust mite extracts can be recovered following the
protection/deprotection sequence.  Optimal
decitraconylation results were found to occur at pH 5.6
and ambient temperature.

TABLE 3

Citraconylation and decitraconylation of house dust mite extracts (D.Pteronyssinus).

| Material* | Citraconic Anhydride (μl) | Prior to treatment with acid | | | Post deacylation | | |
|---|---|---|---|---|---|---|---|
| | | $^{o}1NH_2$† μeq/mg | RAST Inhibition≠ (μg/ml) | pH | Temp $^{o}C$ | $^{o}1NH_2$† μeq/mg | RAST Inhibition≠ (μg/ml) |
| HD1≢ | 0 | 0.66 | 0.5 | 5.6 | 23 | 0.76 | 0.69 |
| HD2 | 50 | 0.26 | 25 | 8.0 | 4 | 0.23 | 29 |
| | 50 | 0.26 | 25 | 6.0 | 4 | 0.35 | 5.6 |
| | 50 | 0.26 | 25 | 5.6 | 4 | 0.38 | 3.8 |
| | 50 | 0.26 | 25 | 8.0 | 23 | 0.26 | 31 |
| | 50 | 0.26 | 25 | 6.0 | 23 | 0.53 | 2.7 |
| | 50 | 0.26 | 25 | 5.6 | 23 | 0.59 | 1.4 |
| HD3 | 100 | 0.20 | 60 | – | – | – | – |
| HD4 | 150 | 0.20 | 100 | – | – | – | – |
| HD5 | 200 | 0.20 | 100 | – | – | – | – |
| HD6 | 250 | 0.23 | 150 | – | – | – | – |

† Primary amino content exposed in term of microequivalents per mg

≠ Concentration (μg/ml) required to achieve 50% inhibition of mite extract specific IgE antibody (obtained from house dust mite sensitive individuals) binding to house dust mite extract covalently bonded to paper discs

≢ HD1 treated with solvent alone

Example 4

Cross linking of Protected Timothy Pollen Extract and
Subsequent Deprotection - Product Separation by
Membrane Dialysis

Protected allergen extracts obtained from rye
pollen were prepared as described in Example 2.

The above extracts, having the appropriate degree
of citraconylation (0 or approximately 25% primary
amino group modification), were dissolved in phosphate
buffer (0.5M, pH 8.0) to give a concentration of
20mg/ml. Glutaraldehyde solution was then added with
stirring (50µl, 17.5% w/v) to afford a final reaction
concentration of 0.25% w/v. After a period of 1 hour
at ambient temperature, the reaction mixture was placed
in an Amicon cell (50ml) and diafiltered at 5psi over
an XM 300 membrane; 8 sample volumes (25ml) were passed
through the cell using the phosphate buffer described
above.

The reaction mixture was then removed from the
cell, acidified to pH 6.0 using dilute HCl (3.8M), and
left at ambient temperature for 24 hours. Following
basification to pH 7.4 with dilute NaOH (3M), the
resulting solution was concentrated to 6ml volume using
a PM 10 membrane (10,000 molecular weight cut-off) and
then sterile-filtered (0.45m).

Materials serving as buffer controls preparations,
either with (4e) or without (4a) citraconylation,
received no glutaraldehyde but in all other respects
were treated in like manner to the polymerised samples.

0083497

Samples were taken from the reaction sequence at various points for process control measurements.

Chemical characterisation data for intermediate and final reaction products are collected in Table 4.

Skin test activities for the various preparations, carried out by prick testing in grass pollen sensitive volunteers, are shown in Table 5.

The data shows that protected materials polymerised with glutaraldehyde regain allergenic epitopes following decitraconylation (RAST inhibition data) yet remain substantially reduced in allergenic activity as measured by skin test.

TABLE 4

Chemical Characterisation Data for Polymerised Rye Grass Pollen Extracts

(Example 4)

| Code* | Protection - citraconylated extract≠ | Citraconylated Polymer | | Decitraconylated Polymer | |
|---|---|---|---|---|---|
| | | $^{O}1NH_2$ groups meq/gm | RAST inhibition concentration required to give 50% inhibition (µg/ml) | % substitution $^{O}1NH_2$ groups meq/gm | RAST inhibition concentration required to give 50% inhibition (µg/ml) |
| R4a | 0 | 0.71 | 0.70 | 0.81 | 0.75 |
| R4b | 0 | 0.19 | 400≠ | 0.13 | 360 |
| R4c | 20 | 0.06 | 135 | 0.14 | 19.0 |
| R4d | 20 | 0.05 | 210 | 0.14 | 25.0 |
| R4e | 20 | 0.52 | 2.0 | 0.70 | 1.05 |

\* Samples R4a and R4e represent buffer control materials without and with citraconylation treatment respectively; sample R4b represents a sample polymerised without protection.

≠ Unprotected extract polymerised with glutaraldehyde prior to exposure to decitraconylation conditions.

- 18 -

## TABLE 5

### Skin Test Activities for Polymerised Rye Grass Extracts

(Example 4)

| Volunteer* | Concentration required to achieve threshold response[+] (µg/ml) | | | | | |
| :---: | :---: | :---: | :---: | :---: | :---: | :---: |
| | Test Samples | | | | Ratio polymer: control | |
| | R4a✝ | R4e | R4c | R4d | R4c/R4a | R4d/R4a |
| A4 | 12.5 | 12.5 | 800 | 200 | 64 | 16 |
| B4 | 25 | 25 | >800 | 800 | >32 | 32 |
| C4 | 50 | 100 | >800 | >800 | >16 | >16 |
| D4 | 6.3 | 3.1 | 400 | 800 | 64 | 128 |
| E4 | 12.5 | 12.5 | 800 | 400 | 64 | 32 |

+ End point taken at 20mm$^2$ wheal area

* Grass pollen sensitive volunteers

✝ See table 4 for test sample coding

EXAMPLE 5

Preparation of polymers of Timothy grass pollen extract
with varying molecular weigh distribution

Protected allergen extracts obtained from Timothy
pollen were prepared as described in Example 2.

The above extracts, having varying degrees of
citraconylation (within the range 0 - 50% primary amino
modification) were dissolved in phosphate buffer (0.5M,
pH 8.0) at concentrations between 20 - 70mg/ml.

Glutaraldehyde modification and subsequent
separation of polymeric products were prepared as
described in Example 4.

Chemical characterisation data for final reaction
products are given in Table 6.  Gel elution profiles
for the various products illustrated using Sepharose 4B
that increased molecular weight materials were produced
when higher concentration of extracts were employed in
the polymerisation reaction.

Skin test activities for the various preparations,
carried out by prick-testing in grass pollen sensitive
volunteers, are shown in Table 7.

The data illustrated that protected materials,
polymerised with glutaraldehyde at increasing extract
concentrations, manifest corresponding increases in
molecular weight distribution and are markedly reduced
in allergenic activity.

TABLE 6

Chemical Characterisation for Polymerised Timothy Pollen Extracts (Example 5)

| Coding | Protection (citraconylated extract)[†] | Extract concentration used for polymerisation (mg/ml) | Citraconylated polymer $^{o}_{1}NH_2$ groups (meq/gm) | Decitraconylated polymer | |
|---|---|---|---|---|---|
| | | | | $^{o}_{1}NH_2$ groups (meq/gm) | RAST Inhibition[‡] (µg/ml) |
| T5a[≠] | 0 | 20 | – | 0.64 | 0.95[a] |
| | | 20 | | | 0.43[b] |
| T5b | 0 | 20 | 0.12 | 0.12 | 270[a] |
| T5c | 23 | 20 | 0.09 | 0.16 | 90[a] |
| T5d | 23 | 70 | 0.06 | 0.15 | 65.0[b] |

[†]  $^{o}_{1}NH_2$ groups treated with citraconic anhydride

[≠]  Buffer control material

[‡]  Concentration of extraction required for 50% inhibition; superfixes a and b refer to independent assays.

a,b  RAST assays carried out at different times

## TABLE 7

### Skin Test Activities for Polymerised Timothy Grass Extract

#### (Example 5)

| Volunteer* | Concentration required to achieve threshold response≠ (µg/ml) | | | | | | |
| --- | --- | --- | --- | --- | --- | --- | --- |
| | Test samples | | | | Ratio polymer : control | | |
| | T5a≠ | T5b | T5c | T5d | T5b/T5a | T5c/T5a | T5d/T5a |
| A5 | 12.5 | 800 | 800 | 800 | 64 | 64 | 64 |
| B5 | 6.3 | 400 | 100 | 400 | 64 | 16 | 64 |
| C5 | 12.5 | >1600 | 800 | 1600 | >128 | 64 | 128 |
| D5 | 25 | >1600 | >1600 | >1600 | >64 | >64 | >64 |
| E5 | 100 | >1600 | >1600 | >1600 | >16 | >16 | >16 |

≠ End point taken at 20mm$^2$ wheal area

* Grass pollen sensitive volunteers

≠ See table 6 for test sample coding

EXAMPLE 6

Cross-linking of Protected Grass Pollen Allergens and Subsequent Deprotection: - Product separation by gel elution chromatograph

Protected allergen extracts obtained from Timothy, rye and cocksfoot pollents were prepared as described in Example 2. The following general procedure was then applied to each extract. Citraconylated materials (approximately 20-60% primary amino group modification) were dissolved in phosphate buffer (0.5M, pH 7.0) at 20mg/ml and stirred at ambient temperature during the addition of glutaraldehyde solution (final concentration 0.25% w/v). After a further 1 hour period, each polymerised sample was applied to a gel filtration column (Sephacryl S200) and eluted with PBS (0.01M, pH 7.4); excluded fractions were collected and pooled.

Materials serving as buffer control preparations received no glutaraldehyde nor fractionation step but were diluted to the same volume as the excluded fractions and allowed to stand at room temperature for four hours at pH 8.0; these conditions were equivalent to the fractionation step.

To each pooled fraction or corresponding control solution was added phosphate buffer (1ml, 0.5M, pH 5.2) and the pH was adjusted to 5.2 with dilute HCl (3.8M); the solutions were then incubated for an overnight period at 30-37°C. Following basification to pH 8.0 with dilute sodium hydroxide (3M), the materials were dialysed extensively against Evans solution (pH 7.4) and finally filtered through a millipore membrane (0.8μ).

Chemical characterisation data for the various preparations are collected in Table 8.

Skin test activities of these same preparations, carried out by prick test in pollen sensitive individuals, are given in Tables 9 to 11.

The capacity for polymerised extracts to induce allergen extract specific antibody was assessed by immunisation studies in rats.

Male Wistar rats (in groups of 5) were treated with polymerised grass pollen extracts as above, or extract which had received the acylation/deacylation treatment without glutaraldehyde exposure, in Freund's complete adjuvant. Each animal received 100μg of the appropriate grass pollen extract in 1ml of vehicle at two subcutaneous sites. All animals were bled on days 28 and 35 after the initial treatment; on day 28 all groups received an additional subcutaneous booster dose of the respective immunogen (50μg) in an aqueous vehicle. Antibody data measured by passive haemagglutination are shown in Table 12.

The data shows that antibody responses for materials polymerised with protection were markedly reduced in allergenicity yet retained the capacity to produce allergen extract specific antibody.

Table 8

Chemical Characterisation Data for Protected Polymerised
Grass Pollen Extracts (Example 6)

| Extract | Code | % Protection: citraconylated extract ✝ | Citraconylated polymer $^{o}1\ NH_2$ meq/gm | Decitraconylated polymer | |
|---|---|---|---|---|---|
| | | | | $^{o}1\ NH_2$ meq/gm | RAST ✝ inhibition (µg/ml) |
| Timothy | T6a✝ | O | 0.54 | 0.56 | 0.7 |
| | T6b | 20 | 0.07 | 0.24 | 33 |
| | T6c | 58 | 0.07 | 0.41 | 18 |
| | T6d✝ | O | 0.47 | 0.51 | 0.8 |
| | T6e | 20 | 0.08 | 0.13 | 51 |
| | T6f | 58 | 0.02 | 0.22 | 40 |
| Rye | R6a✝ | O | 0.58 | 0.62 | 0.2 |
| | R6b | 22 | 0.06 | 0.12 | 11 |
| | R6c | 60 | 0.03 | 0.31 | 4 |
| | R6d✝ | O | 0.59 | 0.61 | 1.4 |
| | R6e | 22 | 0.03 | 0.11 | 53 |
| | R6f | 60 | 0.01 | 0.43 | 30 |
| Cocksfoot | C6a✝ | O | 0.65 | 0.48 | 0.6 |
| | C6b | 24 | 0.03 | 0.13 | 42 |
| | C6c | 62 | <0.01 | 0.33 | 21 |
| | C6d✝ | O | 0.65 | 0.48 | 1.5 |
| | C6e | 24 | 0.03 | 0.11 | 40 |
| | C6f | 62 | <0.01 | 0.33 | 24 |

✝ $^{o}1\ NH_2$ group treated with citraconic anhydride

✝ concentration (µg/ml) required to achieve 50% inhibition of human grass pollen extract specific IgE uptake on to grass pollen extract covalently bonded to paper discs

✝ buffer control materials

Table 9

Skin Test Activities for Polymerised Timothy Grass Pollen
Extracts (Example 6)

| Volunteer* | Concentration required to achieve threshold response✝ (µg/ml) | | | | | | | | | |
|---|---|---|---|---|---|---|---|---|---|---|
| | Test Samples | | | | | | Ratio polymer:control | | | |
| | T6a✝ | T6b | T6c | T6d | T6e | T6f | $\frac{T6b}{T6a}$ | $\frac{T6c}{T6a}$ | $\frac{T6e}{T6d}$ | $\frac{T6f}{T6d}$ |
| A3 | 3.1 | 100 | – | 12.5 | 100 | – | 32 | – | 8 | – |
| B3 | 3.1 | 100 | – | 6.3 | 50 | – | 32 | – | 8 | – |
| C3 | 12.5 | 400 | – | 25 | 200 | – | 32 | – | 8 | – |
| D3 | 25 | – | 400 | 100 | – | 400 | – | 16 | – | 4 |
| E3 | 25 | – | 100 | 12.5 | – | 200 | – | 4 | – | 16 |
| F3 | 12.5 | – | 100 | 12.5 | – | 200 | – | 8 | – | 16 |

✝ End point taken as 20mm$^2$ wheal area

* Grass pollen sensitive volunteer

✝ See Table 8 for test sample coding

Table 10

Skin Test Activities for Polymerised Rye Grass Pollen
Extracts (Example 6)

| Volunteer* | Concentration required to achieve threshold response[†] (μg/ml) | | | | | | | | | |
|---|---|---|---|---|---|---|---|---|---|---|
| | Test Samples | | | | | | Ratio Test:control | | | |
| | R6a[†] | R6b | R6c | R6d | R6e | R6f | $\frac{R6b}{R6a}$ | $\frac{R6d}{R6a}$ | $\frac{R6e}{R6d}$ | $\frac{R6f}{R6d}$ |
| A | 1.6 | 400 | – | 12.5 | >400 | – | 256 | – | >32 | – |
| B | 1.6 | >400 | – | 12.5 | >400 | – | >256 | – | >32 | – |
| C | 12.5 | 400 | – | 12.5 | 400 | – | 32 | – | 32 | – |
| D | 12.5 | – | 200 | 50 | – | 400 | – | 16 | – | 8 |
| E | 25 | – | 100 | 25 | – | 400 | – | 4 | – | 16 |
| F | 3.1 | – | 50 | 12.5 | – | 400 | – | 16 | – | 32 |

† End point taken as wheal area

\* Grass pollen senstive volunteer

† See Table 8 for test sample coding

Table 11

Skin Test Activities for Polymerised Cocksfoot Grass
Pollen Extracts (Example 6)

| Volunteer* | Concentration required to achieve threshold response[†] ($\mu$g/ml) | | | | | | | | | |
| --- | --- | --- | --- | --- | --- | --- | --- | --- | --- | --- |
| | Test Samples | | | | | | Ratio Test:control | | | |
| | C6a[†] | C6b | C6c | C6d | C6e | C6f | $\frac{C6b}{C6a}$ | $\frac{C6c}{C6a}$ | $\frac{C6e}{C6d}$ | $\frac{C6f}{C6d}$ |
| A | 6.3 | 200 | – | 6.3 | 400 | – | 32 | – | 64 | – |
| B | 12.5 | 200 | – | 12.5 | 400 | – | 16 | – | 32 | – |
| C | 12.5 | 400 | – | 6.3 | >400 | – | 32 | – | >64 | – |
| D | 50 | – | >400 | 25 | – | >400 | – | >8 | – | >16 |
| E | 50 | – | 400 | 25 | – | >400 | – | 8 | – | >16 |
| F | 6.3 | – | 400 | 6.3 | – | 100 | – | 64 | – | 16 |

/  End point taken as 20mm$^2$ wheal area

*  Grass pollen sensitive volunteer

/  See Table 8 for test sample coding

Table 12

Immunogenicity of Polymerised Extracts in Rats (Example 6)

| Material | Serum Antibody by Tanner Cell Agglutination $\neq$ Titre as $-\log_2$ from $\frac{1}{2}$ | | | | | |
| | Day 28 | | | Day 35 | | |
| | T$\neq$ | R | C | T | R | C |
| Buffer control** | ND* | 9 | 9 | ND | 14 | 15 |
| | ND | 10 | 10 | + | 15 | 15 |
| | + | 10 | 8 | 10 | 14 | 14 |
| | ND | 11 | 8 | + | 14 | 17 |
| | +* | 11 | 7 | ND | 15 | 13 |
| Polymerised with protection and deprotection** | 6 | 6 | 8 | 8 | 10 | 13 |
| | 7 | 8 | 8 | 10 | 10 | 13 |
| | 9 | 8 | + | >12 | 8 | 12 |
| | 8 | 10 | 5 | 9 | 12 | 12 |
| | ND | 9 | 9 | + | 13 | 11 |

$\neq$  Grass extract employed

$\neq$ : + - Detectable but weak response

*  : ND - Non-detectable agglutination

**  Test samples coded as buffer control or 20% protected
    (primary amino group)

$\neq$  Data given for each animal in treatment group.

0083497

1.  A process for the preparation of a modified allergen, which comprises protecting a substantial proportion of the allergenic sites, reacting the allergen so protected with a cross-linking agent to give a linked product of substantially enhanced molecular weight distribution, and then deprotecting the linked product.

2.  A process according to claim 1, in which the allergen comprises a purified extract of pollen or house dust.

3.  A process according to claim 1 or claim 2, in which at least 10% of chemical functional groups on the allergen are left unprotected.

4.  A process according to any one of claims 1 to 3, in which the allergen is protected by treatment with an unsaturated dicarboxylic acid anhydride.

5.  A process according to any one of claims 1 to 4, in which the cross-linking agent comprises a dialdehyde.

6.  A modified allergen whenever prepared by a process according to any one of claims 1 to 5.

7.  A pharmaceutical composition comprising a modified allergen according to claim 6 and a pharmaceutically acceptable carrier.

8.  A composition according to claim 7 in the form of an injectable desensitisation vaccine.